(19) **Europäisches Patentamt / European Patent Office / Office européen des brevets**

(11) **EP 3 187 200 B1**

(12) **EUROPEAN PATENT SPECIFICATION**

(45) Date of publication and mention
of the grant of the patent:
**07.07.2021 Bulletin 2021/27**

(51) Int Cl.:
*A61M 1/00* *(2006.01)*          *A61F 13/00* *(2006.01)*
*A61F 13/02* *(2006.01)*

(21) Application number: **15203107.6**

(22) Date of filing: **30.12.2015**

(54) **PORTABLE NEGATIVE PRESSURE DEVICE**

TRAGBARE UNTERDRUCKVORRICHTUNG

DISPOSITIF DE PRESSION NÉGATIVE PORTABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.07.2017 Bulletin 2017/27**

(73) Proprietor: **Paul Hartmann AG
89522 Heidenheim (DE)**

(72) Inventors:
• **Klusmann, Johanna
  89522 Heidenheim (DE)**
• **Beyrle, Karina
  89522 Heidenheim (DE)**
• **Eckstein, Axel
  89522 Heidenheim (DE)**

• **Hofstetter, Jürgen
  89522 Heidenheim (DE)**
• **Croizat, Pierre
  89522 Heidenheim (DE)**
• **Hsieh, Mark
  Cambridge, CB1 2EF (GB)**
• **Stein, James
  Cambridge, CB21 5DX (GB)**
• **Dawber, Chris
  Cambridge, CB1 9PQ (GB)**

(56) References cited:
EP-B1- 0 777 504          WO-A1-02/093272
US-A1- 2010 305 523       US-A1- 2015 025 485
US-A1- 2015 073 338

## Description

FIELD OF THE INVENTION

The disclosure relates to a portable negative pressure wound therapy device that can be carried on the body of a user for providing a vacuum for medical vacuum treatment of wounds on the body of a person or of an animal, comprising an electrically actuated suction pump for draining wound fluids from a patient, a programmable electronic control device (herein termed as a "microelectronic controller"), which controls the suction pump, an electronic memory, which is required for storing information such as operating instructions for the controller and a housing for containing the electrical and/or electronic components of the device. Typically, a negative pressure wound therapy device of this type comprises a user interface, such as a touch-screen display, adapted to receive user settings and/or to display one or more system conditions.

The above-mentioned expression "portable device" means that the patient can carry the device along so that he/she is mobile and his/her wound can nevertheless be permanently treated, i.e. without interruption. The portable device may thereby be held on the body of the patient and be carried along by means of any fastening means, for example in the form of a flexible belt or a shoulder strap. A portable device of the above-mentioned type may naturally also be used for stationary operation, i.e. detached from the body of the patient. In this case, it may e.g. be mounted to a hospital bed or be deposited next to the hospital bed.

DESCRIPTION OF THE PRIOR ART

Vacuum wound treatment devices have been described many times, in particular, in US 2004/0073151 A1, WO 2009/047524 A2, EP 1 905 465 A1, WO 2008/039314 A2 or EP 777 504 B1 as well as in EP 1 863 549 B1, EP 2 464 394 A1, WO 2012/156174 A1 or EP 2 464 393 A1 of the assignee.

EP-0777504-B1 relates to a negative pressure wound treatment device, which comprises a canister fill sensor. Preferably the fill sensor is a capacitive sensor.

US-20150025485-A1 discloses a negative pressure wound treatment system having an orientation detection device coupled to a sidewall of the canister. The orientation detection device may be a motion detection device, an accelerometer or a gyroscope.

US-20100305523-A1 describes a portable negative pressure wound treatment system comprising a microelectronic control unit, a memory module and a tip sensor, such as a for example a four position angle sensor, to detect a change in an orientation of the canister assembly.

WO-2002/093272-A1 discloses an electronic movement monitor device (MMD) which is provided to be attached to a person or to an object. The document suggests, that the MMD may also be attached to a medical device to detect and store impacts acting on the device during transport. The MMP may be adjusted to determine impact events that exceed a predetermined threshold.

US 2015/073338 A1 discloses a pump used to deliver fluids to a subject, the pump having a sensor for detecting movement of the pump and configured to detect both an acceleration sequence indicative of the pump being used while the subject is ambulating, and a critical impact to which the device was exposed.

In devices of this type for vacuum treatment of wounds, a suction pump (sometimes incorrectly called "vacuum pump") communicates with the wound or the wound area via a suction line, wherein a wound dressing with an air-tight cover material is provided for air-tight sealing of the wound and the wound area, such that a vacuum can be generated in the wound region and fluids can be extracted by suction from the wound region.

The term vacuum in connection with the present invention defines an air pressure that is lower than the ambient air pressure (atmospheric air pressure), in particular, inside a wound dressing. The cover material of a wound dressing for air-tight sealing of a wound region must therefore be designed in such a fashion that it withstands the pressure difference that is established such that a vacuum can actually be applied to and maintained in the wound region. The wound dressing and the cover material are, however, typically flexible to a certain degree. In the field of vacuum therapy for the treatment of wounds, the vacuum is quantitatively defined as the pressure difference between ambient air pressure and the air pressure applied below the cover material. In the field of vacuum therapy, this pressure difference is typically at most 250 mmHg (mm mercury column) (1 mm Hg=1 Torr =133.322 Pa). This vacuum range of up to maximally 250 mmHg has turned out to be suitable for wound healing. A preferred vacuum range is between 10 and 150 mmHg.

For typical vacuum treatment, the vacuum that is applied to the wound using the device can either be kept substantially constant with time or can be varied with time, in particular, in cycles, which can be realized by a correspondingly designed and programmed control device for the vacuum-generating device, in particular, in dependence on further parameters.

An advantageously flexible suction line, e.g. in the form of a drainage hose, is provided for applying a vacuum and advantageously also for extracting body fluids, the drainage hose communicating at one end with the wound area or the wound region via a so-called port in the area of the wound cover material, and at the other end communicating with a container for receiving the sucked body fluids, or with the vacuum generating device.

In addition to vacuum wound treatment, the present device may also be used for other applications for providing a vacuum for medical applications, in particular, extraction of any body fluids by suction, in the field of medical incontinence management, in the field of care of stoma patients or in the field of extraction of wound exudates, if necessary, thereby using rinsing liquids and also without application of a vacuum over considerable time periods.

## SUMMARY OF THE INVENTION

In contrast to a stationary medical device, a portable medical treatment device may frequently be exposed to a changing environment. The device can be used even outside of a medical facility, for example during travel or in a home environment. Any unforeseen malfunction of the device may lead to an interruption of the therapy, which can possibly cause harm to the patient. Based on a portable device of this type for providing a vacuum for medical applications, it is the underlying purpose of the present invention to further optimize the user friendliness and operational safety of the device. The medical device should be as reliable and as fail-proof as possible. Ideally, even a technically less skilled user or patient should be given the feeling that she/he can safely control operation.

In accordance with the invention, this object is achieved with a method according to claim 1. The method includes using a device of the above-mentioned type in that at least one microelectronic impact sensor is provided for determining, if an impact acting on the device is present.

In accordance with the invention a portable negative pressure wound therapy device is provided. The device is adapted to be carried by a user. The device comprises

- an electrically actuated suction pump for draining wound fluids from a patient,

- at least one microelectronic controller,

- at least one electronic memory,

- a housing for containing the electrical and/or electronic components.

The device is characterised in that the device further comprises at least one microelectronic impact sensor, wherein the impact sensor is adapted to detect an impact acting on the device. In particular, the microelectronic impact sensor is adapted to detect acceleration, rotation, air humidity, atmospheric air pressure, temperature and magnetic field strength impacts. Accordingly, the at least one impact sensor is one or more selected from the group of linear acceleration sensors, gyration sensors, humidity sensors, temperature sensors, magnetic field sensors and atmospheric air pressure sensor.

According to the invention, the at least one impact sensor interacts with at least one microelectronic controller and/or with at least one electronic memory. The microelectronic controller is a component of the control unit. The control unit is the central instance for controlling and monitoring function of the device. Accordingly, when the at least one impact sensor interacts with the microelectronic controller it is also interacting with the control unit.

Interaction of the impact sensor with the microelectronic controller may include analysis of the raw data produced by the sensor and selection of the data to be recorded on an electronic memory. Interaction between the impact sensor and the microelectronic controller may, for example, result in the display of warning messages or signals to the user.

During inspection of malfunctioning portable negative pressure wound therapy devices, the inventors of the present invention found, that at least 50 % of the technical defects or malfunctions observed were most likely related to external impacts that were acting on the devices. The presumptive harmful impacts included crashes, exposure to humidity or moisture and extreme temperatures. However, in most such cases no signs of impact were visible when inspecting the outside of the housing of the devices.

The harmful impacts include crashes, which did not leave any visible traces on the housing of the device. However, such crashes can, for example, loosen electronic contacts or untighten mechanical connections, such as tube connections.

Another significant class of impacts include exposure to humidity, which may interfere with electronic components. Exposure to harmful levels of humidity do not necessarily leave any visible damages, but may nevertheless interfere with proper function of the device.

Exposure to extreme temperatures includes exposure to very high temperatures (e.g. more than 50 °C) or to very low temperatures (e.g. less than 0°C). Such exposure can, for example, occur if the device is left in a car for an extended period of time. Very high temperatures can lead to a malfunction of electric/electronic components (for example by weakening electronic contacts or by damaging electronic circuits) as well as to a malfunction of mechanical components (for example by causing leakages to tube connectors). Exposure to very low temperatures can entail humidity (condensation water) when the device is warmed up again. Very low temperatures can also affect integrity of the plastic conduits.

Strong magnetic fields, which can for example appear in close neighbourhood to medical NMR-devices in hospitals, may cause damages to the electronic memory. Exposure to strong magnetic fields does not leave any visible damages.

In some rare cases, it was also suspected that malfunctions of the control electronic may be related to exposure to varying atmospheric pressure, which may for example occur during a flight. The latter case does not leave any physical damages to the device, so it is particularly difficult for a service technician to evaluate the reason for a malfunction being reported by the user and/or by the error messages stored in the history of the control system.

Impacts of the type, which were exemplified above, can lead to immediate malfunction of the medical device. In an up-to-date device a malfunction is usually detected by self-control algorithms, which are permanently running to monitor proper function of the device. However, the self-control algorithm is only able to recognise the fact that the device is not working properly. It is usually not able to identify the cause of a malfunction, if the malfunction was generated by an external impact acting on the device.

Moreover, impacts of the type, which were exemplified above, can also lead to a stress on the device, which does not entail immediate malfunction, but which can lead to a higher probability that a malfunction will appear in the future. In some cases it will not be possible to detect any visible traces of a past external impact, even if the device is carefully inspected.

The impact sensor of the present invention can detect an external impact acting on the portable negative pressure wound therapy device. The information obtained by the impact sensor can advantageously be correlated to an existing malfunction of the device.

Also, the information obtained by the impact sensor can advantageously be used to inform a user that an impact acting on the device has occurred, even if no malfunction of the device is present. In this case the user may, for example, be warned, that a possible malfunction related to the impact may occur in the future (even if no malfunction of the device is currently appearing). Advantageously, the device may be locked, if an impact exceeding a certain strength has acted on the device. A locking of the device may be implemented to retain a user from using a device for therapy, which may carry a potential (but not visible) damage related to an external impact.

It is also possible to use the information obtained from the impact sensor for maintenance purposes. In a (non-limiting) example a service technician can use the information obtained from the impact sensor to selectively inspect or exchange components, which are typically prone to deficiency when exposed to certain kinds of external impacts. The technician may, for example, selectively exchange the main control board of the device, if an impact sensor, which can detect high levels of humidity, has determined exposure to high levels of humidity in the past. As another example, it may also be possible, that a technician inspects tightness of conduit connections, if an impacts sensor, which can detect high levels of acceleration, has determined a crash condition in the past. As known to a person skilled in the art, many other specific maintenance actions can be performed, once the nature and optionally the strength of the external impact has been determined by the impact sensor.

Advantageously, when making use of the present invention, a patient using the device as well as a caregiver (such as a nurse) using the device, will be warned, if an external impact may affect proper functionality and integrity of the treatment device.

It is a particular advantage of the present invention, that impacts, which were acting on the device in the past and which did not leave any visible traces, can be detected and/or recorded.

It is another significant advantage of a preferred embodiment of the present invention, that even such impacts, which were acting on the device while the device was switched off, can be detected and/or recorded.

DESCRIPTION OF THE PREFERRED EMBODIMENTS

According to a most preferred embodiment of the invention, the portable negative pressure wound therapy device comprises at least one linear acceleration sensor. A linear acceleration sensor is able to detect any kind of crashes, hits, flips or downthrows that the device is subjected to. Preferably, the at least one linear acceleration sensor should be capable of detecting acceleration in any of the three axis of space ($a_x$; $a_y$; $a_z$). It is particularly preferred that the linear acceleration sensor is a component of a microelectromechanical system (MEMS) integrated in a chip. One example well suited for the invention is the three channel MEMS motion sensor H3LIS331DL from ST Microelectronics, United Kingdom, which can detect acceleration along each of the three axes of space of up to 400 g.

When using an acceleration sensor, which is capable of detection acceleration in any of the three axes of space ($a_x$; $a_y$; $a_z$), it has been found advantageous to calculate the magnitude $M_\alpha$ using the formula:

$$M_\alpha = \sqrt{(a_x^2 + a_y^2 + a_z^2)}$$

Same as for acceleration, the absolute dimension for $M_\alpha$ is m/s². For the purposes of the present invention, for example to set thresholds, it is also possible to use a relative unit (i.e. a value without dimensions) for $M_\alpha$. A relative value for $M_\alpha$ can - for example - be generated by entering the value corresponding to the output of each acceleration sensor channel into the aforementioned formula.

Magnitude $M_\alpha$ reflects the overall strength of linear acceleration stress acting on the device.

According to another preferred embodiment, the negative pressure wound therapy device is adapted to record the impact sensor data on the at least one memory. In principle, it is possible to record the complete output of the impact sensor (i.e. the raw data). Due to the high sampling rate of a typical impact sensor, such as an acceleration sensor (an acceleration sensor has a typical sampling rate of 1000 Hz), storage of the produced raw data will lead to extensive consumption of memory space. It is therefore preferred, that the raw data are only stored for a short period of time, such as a time window of, for example, 5 seconds, to enable the microelectronic controller to analyse the data. After analysis, the raw data are overwritten and only the result of the analysis is stored for an extended period of time. According to another preferred method to further save memory space, the storing of impact sensor related data (i.e. impact sensor raw data or information related to the result of an analysis of impact sensor raw data) is done only under the proviso that the strength of the impact is above a predetermined level. A device according to this preferred embodiment is adapted to record an impact, if the impact is above a predetermined threshold level. The expression "to record an impact" includes storing the sensor raw data on an electronic memory component, such as a flash memory. The expression "to record an impact" also includes storing any information or results that an algorithm running on a controller deduces from the impact sensor raw data. Saving only the results of an evaluation is the most effective way to save memory. Additional saving of memory space can be achieved by applying standard methods of data compression know in the art.

In general, the predetermined threshold can conveniently be selected in sufficient distance to the level, which is correlated to the certified tolerance of the device for the specific external impact. For example, if the device, according to the specification of the manufacturer, is destined to be stored within a temperature range between -25°C and 70°C, the thresholds for temperature could be -20°C (near lower threshold, i.e. recording of temperature will start, if temperature falls below -20°C) and 65°C (near upper threshold, i.e. recording of temperature will start, if temperature is above 65°C). It is also possible to set two separate threshold ranges, i.e. one range for storage (like for example between - 20° and 65°C) and the other range for an active device (for example a lower and upper threshold of 10°C resp. 35°C, if the manufacturer allows the device to be used between 5°C and 40°C).

The threshold for linear acceleration should be correlated to the type of device and to the mechanical stability of the device. The threshold value for linear acceleration should be selected below an acceleration value, where first harmful effects on the device are to be expected. Typically, a drop from 1 m height to a stiff ground will cause acceleration values in the range of 30 g to 150 g. It was found, that an adequate impact threshold for linear acceleration is an acceleration value selected from the range of 3 g to 100 g, in particular 5 g to 50 g, most preferred 10 g.

When measuring acceleration along the three axes of space $(a_x; a_y; a_z)$, it is advantageous to activate recording, if the detected acceleration along at least one of the axes of space exceeds the threshold.

When using a three axis acceleration sensor, it is also possible to use magnitude $M_\alpha$ as a reference parameter. Magnitude $M_\alpha$ is calculated as explained above herein. For example, a threshold selected from an acceleration Magnitude $M_\alpha$ value between 2 m/s$^2$ and 1000 m/s$^2$ can be used. However, the threshold has to be selected for each type of device individually, taking into consideration the specific nature of the device with regard to stability, form, size, weight and the like.

An adequate impact threshold for gyration is selected from the range of 2 dps (degrees per second) to 50 dps, preferable 5 dps. This means recording of gyration will start, as soon as the detected rotation value is higher than the selected threshold.

An adequate lower threshold for exposure to humidity can for example be selected from the range of 80 to 95 % relative humidity, preferable 85 % relative humidity. This means recording of humidity will start, as soon as the relative humidity is higher than the selected threshold.

An adequate threshold for exposure to magnetic fields can for example be in the range of 100 μT to 1000 μT, preferably 200 μT to 500 μT. This means recording of the magnetic field will start, as soon as the field is stronger than the selected threshold.

An adequate lower threshold value for exposure to atmospheric air pressure can advantageously be selected from the range of 600 hPa to 700 hPa. For example, if a value of 650 hPa is selected as threshold, recording of air pressure will start if atmospheric air pressure falls below 650 hPa. An adequate upper threshold value for exposure to atmospheric air pressure can advantageously be selected from the range of 1000 hPa to 1200 hPa. For example, if a value of 1050 hPA is selected as threshold, recording of air pressure will start as soon as the atmospheric air pressure exceeds 1050 hPa. It is particularly preferred to use 650 hPa as the lower threshold and 1050 hPA as the upper threshold.

According to another preferred embodiment, the negative pressure wound therapy device comprises a gyration sensor capable of detecting rotation. A gyration sensor is particularly useful, if combined with a linear acceleration sensor. According to a particularly advantageous embodiment of the present invention the portable negative pressure wound therapy device comprises a linear acceleration sensor capable of detecting acceleration in the three axis of space $(a_x; a_y; a_z)$ and a gyration sensor capable of detecting gyration in the three axis of gyration $(\omega_x; \omega_y; \omega_z)$. Combination of a linear acceleration sensor with gyration sensor allows to improve the information about the quality of an acceleration impact, which is acting on the device. The use of adequate algorithms for sensor data analysis allows (for example) to distinguish a tilt from a drop.

When using a gyration sensor, which is capable of detecting gyration in the three axis of gyration $(\omega_x; \omega_y; \omega_z)$ it has been found to be advantageous to calculate the magnitude $M_\omega$ based on the single angular rates. Similar to the calculation of the Magnitude $M_\alpha$ explained above, magnitude $M_\omega$ can be determined using the formula:

$$M_\omega = \sqrt{(\omega_x^2 + \omega_y^2 + \omega_z^2)}$$

Magnitude $M_\omega$ reflects the overall strength of the gyration the device is subjected to.

Same as for rotation, the absolute dimension for $M_\omega$ is rad/s (the unit dps is used as well; dps stands for degree per second). For the purposes of the present invention, for example to set a threshold, it is also possible to use a relative unit (i.e. a value without dimensions) for $M_\omega$. A relative value for $M_\omega$ can - for example - be generated by entering the value corresponding to the output of each gyration sensor channel into the aforementioned formula.

Preferably, the linear acceleration sensor and the gyration sensor are components of a single microelectronic inertial measurement unit (IMU) integrated in a chip. One example well suited for this embodiment of the invention is the IMU motion sensor LSM9DS0 ("iNEMO inertial module") from ST Microelectronics, United Kingdom. This inertial module device additionally includes a 3D-magnetometer, which can be used, in addition to detection of acceleration and/or rotation, to determine any magnetic fields acting on the device. It is possible to program the controller to display an alarm message, if a certain magnetic field strength is acting on the device. This is of particular relevance, when using the portable negative pressure wound therapy device in a hospital environment, where medical devices generating strong magnetic fields (such as an MRT-device) are used. The magnetic fields could harm the electronic components of the negative pressure wound therapy device. It is also possible that a magnetometer is used as the only impact sensor. Alternatively, a linear acceleration sensor can be used in combination with a magnetometer, without assessing gyration sensor data.

According to a preferred embodiment of the invention, the at least one impact sensor is one or more selected from the group of linear acceleration sensors, gyration sensors, humidity sensors, temperature sensors, magnetic field sensors and atmospheric air pressure sensor. In addition to the combination of linear acceleration sensors and/or gyration sensors with magnetic field sensors other advantageous examples for using a combination of sensors include (but are not limited to): Combination of linear acceleration sensors and/or gyration sensors with a humidity sensor. This combination is particularly useful, because it allows to detect a major class of detrimental impacts that can act on the device. Supplementing the latter combination with a magnetic field sensor is even more advantageous.

According to another preferred embodiment, the negative pressure wound therapy device is equipped with linear acceleration sensors and/or gyration sensors and an air pressure sensor. The air pressure sensor can be used to safeguard, that the device is only used, if the atmospheric air pressure is within the range allowed for by the manufacturer. A humidity sensor can be used in addition.

Likewise, it is suggested that a temperature sensor can be used, either alone or in combination with any of the above mentioned combination of impact sensors.

If feasible, it is also possible to combine all external impact sensor types mentioned herein.

In general, the portable negative pressure wound therapy device comprises at least one mobile electric power supply, such as a rechargeable battery, to power the electrically actuated suction pump and the controller. It is possible, that the same mobile electric power supply also supplies the impact sensor with electric energy. It is however preferred, that the at least one impact sensor comprises a separate power supply, in particular a battery, a rechargeable battery or a capacitor, wherein the separate power supply is independent of a power supply supplying the suction pump and the controller. The separate power supply shall be adapted to safeguard a permanent supply of electric energy to the sensor, even if the battery required to drive the suction pump is empty.

Likewise, the portable negative pressure wound therapy device comprises at least one electronic memory. In general, the electronic memory (as a component of the control unit) is required for storing operating instructions for the microelectronic controller and/or for recording status information related to the function of the device. Moreover, according to this invention, the memory is required to store impact sensor related data. It is possible, that one and the same electronic memory component is adapted to record any impact sensor related data. According to a more preferred embodiment, the portable negative pressure wound therapy device comprises a separate electronic memory, in particular a flash memory, wherein the separate electronic memory is adapted for recording only impact sensor related data. Accordingly, the separate electronic memory is working independent of the memory, which is used by the control unit to store operating instructions.

It is also advantageously, if the portable negative pressure wound therapy device includes a real time clock, whereby the negative pressure wound therapy device is adapted to record the time of an impact detected by the impact sensor. This means that the storing of impact sensor related data (as explained earlier, impact sensor related data include impact sensor raw data or information related to the result of an analysis of impact sensor raw data) is done together with the time derived from the real time clock. According to this embodiment, the sensor related data are provided with a "time stamp". The real time clock is running permanently. It may be powered by a separate power supply, if available.

It is desirable that the portable negative pressure wound therapy device is capable of detecting and recording potentially detrimental impacts, even if the device is switched off. According to this further preferred embodiment, it is possible, for example, to warn a user about potential (though not visible from external inspection) damages of the device that could compromise proper function of the device during therapy. Such a warning could, for example, be displayed it the device is switched on.

A permanent activity of the impact sensor (i.e. even if the main functions of the negative pressure wound therapy device are switched off) may lead to high consumption of electric energy by the sensor. A good solution in order to keep power consumption of the impact sensor within acceptable limits is to include a sleep-to-wakeup function. This means that the impact sensor is running in a power saving mode, if the device is switched off. If the main functions of the negative pressure wound therapy device are switched on, the impact sensor is awake. The same transition from the sleep to the awake modus occurs also, if an impact (such as a crash leading to an acceleration detected by a linear acceleration sensor) above a predetermined wakeup threshold level is detected by the sleeping impact sensor. The power saving mode (during sleeping) is characterised by a lower sampling activity, which requires less electric energy compared to the wake state.

In particular, the portable negative pressure wound therapy device comprises a sleep-to-wakeup function, the sleep-to-wakeup function being characterised by having

- a sleep phase (power saving mode) correlating with a lower sampling rate, the sleep phase being active when the device is switched off,

- a wake phase correlating with a higher sampling rate, the wake phase being active when the device is switched on,

- a sleep-to-wake transition, if the impact detected by the at least one impact sensor is above a predetermined wakeup threshold level, the sleep-to-wake transition working independent of the switching status of the device.

A typical sampling rate for a linear acceleration sensor during the wake phase is between 500 Hz and 2000 Hz. A good compromise between energy consumption and reliable detection of impacts occurring during the sleep phase is to reduce the sampling rate of the linear acceleration sensor to a value between 1 Hz and 50, in particular 10 Hz.

For example, it was found that for a linear acceleration impact an advantageous wakeup threshold level is an acceleration level in the range of 3 g to 50 g. When using an acceleration sensor, which is capable of detection acceleration in any of the three axis of space $(a_x; a_y; a_z)$ it is also possible to use a magnitude $M_\alpha$ to set an adequate threshold, for example using a $M_\alpha$ value selected from the range of 2 m/s$^2$ to 1000 m/s$^2$. Calcu-

lation of magnitude $M_\alpha$ is performed as explained herein above. Alternatively, when using a three axis acceleration sensor, it is also possible to use the highest appearing acceleration level (g-force) on any of the three axis to activate the wakeup modus.

According to a preferred embodiment the sampling rate of the impact sensor can change from a high sampling rate, such as for example 1000 Hz during a negative pressure wound therapy to a low sampling rate, such as for example 0,5 Hz to 50 Hz, preferably 10 Hz during the "sleeping" modus of the sensor (i.e. the device is switched off). The reduction of the sampling rate leads to a significant reduction of power consumption and increases longevity of the battery.

Impact sensor raw data or any information or results that an algorithm running on the controller deduces from the impact sensor data, such as the above mentioned Magnitude $M_\alpha$, can be stored on an internal electronic memory. Alternatively or in addition it is possible to display an alarm message to the user, if an impact detected by the impact sensor is above a first predetermined alarm threshold level. It is also possible to lock the device in order to prevent a potentially defective device from being used for the medical wound therapy. Preferably the lock can only be removed by a service technician. Locking the device prevents a patient from starting a negative pressure wound therapy using a device, which is prone to malfunction as a consequence of an external impact, which has acted on the device previously.

According to another embodiment, it is suggested that the portable negative pressure wound therapy device comprises an interface that allows for transfer of the impact sensor related data to an external computer. Such an interface is usually already present in up-to-date devices, where it is required to update the software, receive error messages collected during therapy and so forth. According to a more advanced version of the device, the portable negative pressure wound therapy device further comprises an additional remote interface, in particular an interface for remote data transfer, for transmitting impact sensor data to a remote device. This feature can be implemented to improve ease of maintenance of the device by the manufacturer. The remote data transfer requires an interface, which is adapter for data transfer, for example via an internet connection. Standard data transfer technology can be used to copy the impact sensor related data from the device to a remote server. The manufacturer can use the impact sensor related data that he receives via the remote interface for improved service.

According to a very favourable embodiment of the invention, the impact sensor is a component of a "stand-alone impact sensor module". The module comprises at least one impact sensor and a further microelectronic controller (i.e. in addition to the microelectronic controller, which is a component of the control unit) cooperating with the impact sensor. The additional microelectronic controller may also be integrated in the impact sensor chip. The module further comprises a data interface, such as a RS232 interface, a memory, such as a flash memory and a real-time clock. The sensor, the interface, the memory and the real-time clock are in direct interaction with the additional microelectronic controller (i.e. with the microelectronic controller of the stand-alone impact sensor module). A separate battery or accumulator supplies electric energy to the components of the module. Most favourably, the module comprises at least one linear acceleration sensor, capable of detecting crashes. In practice, the sensor capable of detecting crashes should comprise a linear acceleration sensor capable of detecting acceleration in the three axis of space $(a_x; a_y; a_z)$.

The disclosure further includes a (not claimed) method of recording an impact condition, to which a negative pressure therapy device was exposed, comprising the steps

- providing a portable negative pressure wound therapy device according to the present invention,
- setting a predetermined impact threshold,
- detecting an impact by means of the impact sensor,
- recording the impact on an electronic memory,
- optionally further comprising the steps of alarming an user and/or locking the negative pressure wound therapy device, if the impact detected by the impact sensor is above a predetermined threshold level.

The disclosure also includes a (not claimed) method of recording an impact condition, to which a negative pressure therapy device was exposed, comprising the steps

- providing a portable negative pressure wound therapy device according to the present invention,
- setting a first predetermined impact threshold,
- setting a second predetermined impact threshold,
- detecting the impact by means of the impact sensor,
- recording the impact, if the impact detected by the impact sensor is above the first predetermined threshold,
- alarming an user and/or locking the negative pressure wound therapy device, if the impact detected by the impact sensor is above the second predetermined threshold, the second predetermined threshold being higher than the first predetermined threshold level.

The impact to be recorded is preferably a linear acceleration and/or a rotation related to a crash or drop of the device.

BRIEF DESCRIPTION OF THE DRAWINGS

Further characteristics, details, and advantages of the invention result from the appended patent claims and from the drawings and the following description of preferred embodiments of the invention. The drawings show:

FIG 1A schematic drawing of a simple negative pressure wound therapy device including the negative pressure bandage applied to a wound of a patient.

FIGS. 2 a to e Different views of a typical portable negative pressure wound therapy device to generate a vacuum for medical applications.

FIG 3A schematic drawing of the piping system and of the electronic components of a negative pressure wound therapy device according to a preferred embodiment of the present invention.

FIG 4A schematic drawing of the structure of the electronic control system and of the electric components of a portable negative pressure wound therapy device according to a preferred embodiment of the invention.

FIG 5A schematic drawing of the structure of a stand-alone impact sensor module according to a preferred embodiment of the invention.

FIG 6 Example of a warning message displayed to the user of a portable negative pressure wound therapy device according to a preferred embodiment of the invention.

FIGS. 7 a to c Acceleration detected during a drop impact test, to which a portable negative pressure wound therapy device according to a preferred embodiment of the invention was subjected.

Acceleration values are given as g-forces.

7 a Impact on bottom front face

7 b Impact on side face

7 c Orientation of the x-axis and the y-axis

FIGS. 8 a to f Acceleration forces detected during different acceleration impacts, to which a portable negative pressure wound therapy device according to a preferred embodiment of the invention was subjected. From the output of the three linear acceleration channels the magnitude $M_\alpha$ was calculated (FIG 8a, b, d, e, f). Rotation magnitude $M_\omega$ was calculated (FIG 8 c) from the gyration sensor recordings made during the same drop experiment also shown in example 8 a. The charts show the values for the magnitudes $M_\alpha$ and $M_\omega$ in relative units.

8 a Acceleration signals recorded during a drop from 1,0 m

8 b Acceleration signals recorded during a "sitting to a chair" movement of a test user

8 c Angular velocity signals recorded during the same drop from 1,0 m as in impact 8 a

8 d Indication of the incidents occurring during the experiment of example 8 a

8 e Acceleration signals recorded while a test user was walking down a stair carrying the portable negative pressure wound therapy device

8 f Acceleration signals recorded while the portable negative pressure wound therapy device was flipped to the front face

## DESCRIPTION OF THE PREFERRED EMBODIMENT

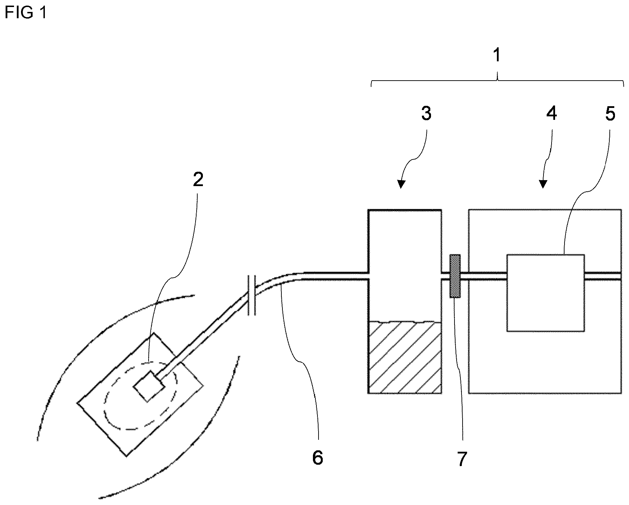

A simple negative pressure wound therapy device 1, which is in fluid communication with a wound 2 of a patient to be treated is shown in FIG 1 schematically. The therapy device 1 comprises a container 3 for collecting the fluids (such as blood, instillation fluids and the like) that are sucked from the wound 2. Wound therapy devices of this type are known in the prior art. The container (or canister) 3 is typically made of a solid material, such as a plastic material. It is usually a disposable article designed for single use. Conveniently, the container 3 can be detachably mounted to the housing part 4 of the device, which contains the electrical components of the apparatus. The container 3 can be evacuated by the electrically actuated suction pump 5. A connection (not shown) is provided for a suction line 6 that leads to the wound such that vacuum communication can be established between the suction pump 5, the container 3, and the suction line 6 that leads to the wound. A filter or air/liquid-separator 7 located within the fluid-pathway between the container 3 and the suction pump 5 is used to prevent exudate from being sucked into the pump 5. A negative pressure wound therapy device typically comprises additional components such as a control system for controlling activity of the pump and means for interacting with the user, such as a touch-screen display or control buttons. These components are not shown in FIG 1.

In some embodiments, the portable negative pressure wound therapy device does not have a container for receiving the drained body fluids. Instead, the body fluids can be contained, for example, in the dressing. This is achieved by providing absorbent layers (not shown in FIG 1). Such negative pressure wound therapy devices, which do not make use of a separate solid exudate canister are typically used for treating less exudating wounds, for example surgical wounds.

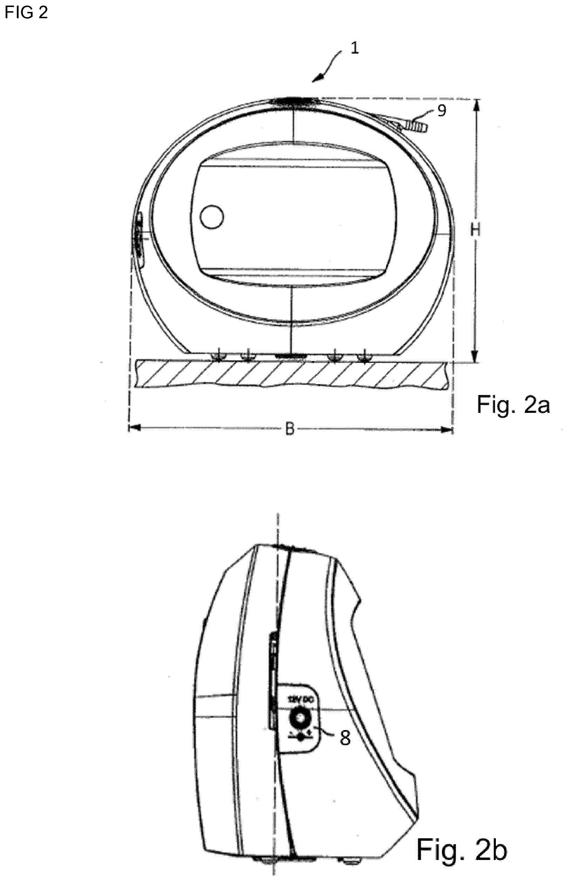

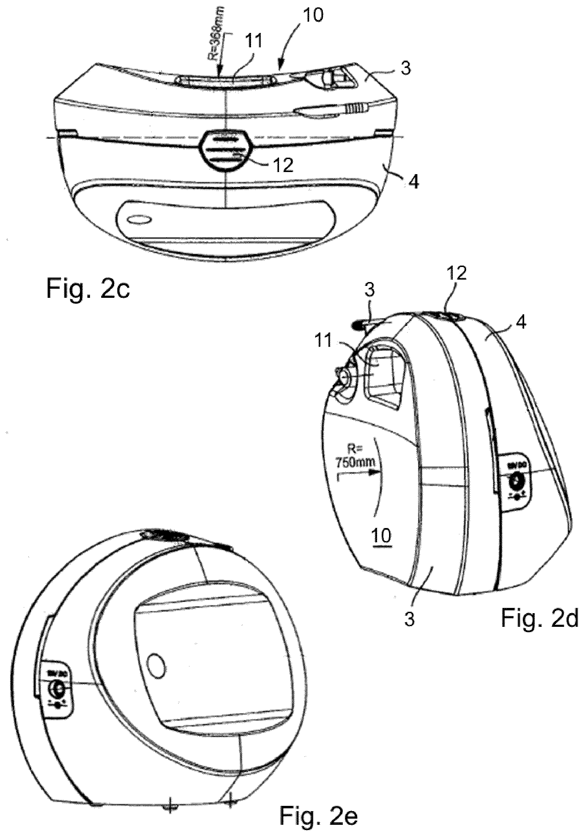

FIGS. 2 a to e show a typical example of a portable device 1 for the provision of the vacuum for medical applications. The device 1 comprises a first housing part 4 in which a vacuum-producing device in the form of an air suction pump as well as the electrical components for the device are accommodated, including batteries or preferably rechargeable batteries. A recharging connection for the batteries is designated by reference symbol 8. Moreover, the device 1 comprises a second housing part in the form of a container 3 for receiving body fluids, in particular, for receiving wound exudates suctioned away from a wound. Preferably, the entire second housing part is constituted as a disposable single-use item. In its upper region, a connection gland 9 for a suction tube is provided that can, for example, lead to a wound dressing sealing the wound when the device 1 is used in the vacuum therapy of wounds and there it may, for example, communicate with the wound space through a port to apply and maintain a vacuum to the wound space and to suction away wound exudates into the container. For this purpose, the container 3 communicates with the suction pump 5.

It can also be seen from FIG 2d on the side 10 of the second housing part 3 facing the body, a grip recess 11 is formed in the shape of an opening extending right through the second housing part 3. In this way, the device 1, or only its second housing part 3, can be gripped and handled with one hand.

In the preferred embodiment shown, a manually operable element 12 is provided in this grip recess 11 on the upper side of the device 1, for example, in the form of a pushbutton that acts on locking and back-gripping means (not shown). In the joined condition of the two housing parts 3 and 4, the locking or back-gripping means are in a locked condition holding the two housing parts 3, 4 together by positive action. Only upon operation of the operating element 12, the lock is released so that the housing parts 3, 4 can be separated from each other.

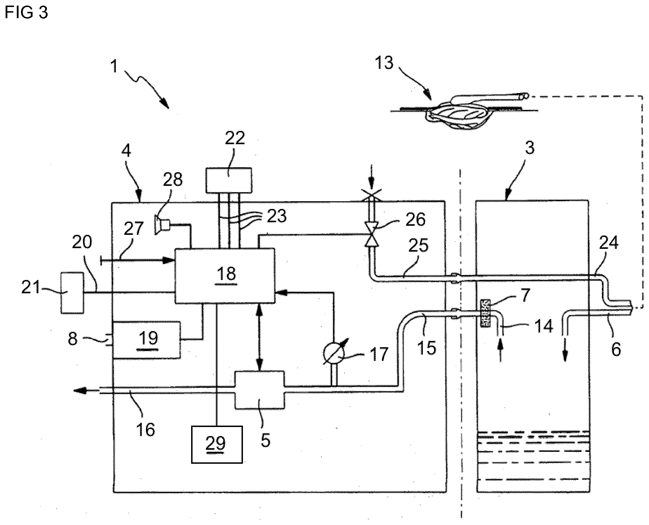

FIG. 3 shows the nature of the piping system and of the electronic components of a negative pressure wound therapy device according to a preferred embodiment of the invention. According to this preferred embodiment, the device comprises an inventive microelectronic impact sensor, wherein the impact sensor is adapted to detect an acceleration impact acting on the device. Apart from the inventive feature, the device is similar to negative pressure wound therapy device of the type exemplified in FIG 2. In contrast to the very basic system shown in FIG 1, the device of FIG 3 includes additional components (known from the art) such as the air rinsing pathway of the fluid system. Based on FIG 3 one embodiment of the inventive device is explained. FIG 3 shows the previously described or a similar device for providing a vacuum for medical applications in a purely schematic representation, wherein relevant reference symbols are used for the corresponding components. However, FIG 3 shows only the components that are relevant for the following description. FIG 3 depicts a schematically indicated wound to be treated with a vacuum with a vacuum-tight wound dressing 13, to which the suction tube 6 emanating from the container 3 leads. From the container 3, a further tube section 14 leads outwardly through the already mentioned filter 7. If the container 3 or the first housing part 4 is put into its operating position on the first or basic housing part 4 of the device 1, the tube section 14 is connected to a further tube section 15 within the first housing part 4 that leads to the intake side of the suction pump 5. When the suction pump 5 operates, a vacuum is applied to the container 3 and to the suction tube 6 via tube sections 14, 15.

Moreover, a pressure sensor 17 for measuring the pressure is provided in the tube section 15 between container 3 and suction pump 5. Its signals are sent to an electronic control unit, collectively identified by reference symbol 18, which performs open-loop and closed-loop control of the device 1 in total. The electronic control unit 18 comprises a microelectronic controller and at least one electronic memory. Also shown is the charging connection 8 for rechargeable batteries that are located in a compartment 19 and a connection 20 for a schematically indicated power supply unit 21. Reference symbol 22 indicates a display unit, preferably having a capacitive switch membrane (touchscreen). A user may control operation of the device via said touchscreen. The electrical connection to the electronic control unit 18 is indicated via electrical lines 23. The suction pump 5 is controlled by the electronic control unit 18 in which, by means of the signals of the pressure sensor 17, a pressure and vacuum closed-loop control is implemented with known open-loop and closed-loop control mechanisms (setpoint/actual value control mechanisms), so that the pressure value corresponding to the currently selected program is controlled in the tube section 15.

Also shown is an additional rinsing or aeration tube 24 that only shown by way of example, leads through the container 3 and just like the suction tube 6 leads to the wound dressing 13. When the container 3 is attached in its intended assembly position on the first housing part 4, this rinsing tube 24 communicates with a tube section 25 provided in the first housing part 4 in which an electromagnetically operated valve 26 is provided that can be actuated by the electronic control unit 18 and connects the tube section 25 with the atmospheric air when it is open, so that an air current toward the wound via the rinsing tube 24 can be generated.

The device 1 and its electronic control unit 18 also feature a data interface 27, preferably a USB interface, by means of which the electronic control unit 18 or its method of operation can be programmed. In addition, device 1 comprises a speaker 28 which is connected to the control unit 18. The speaker can be used to generate acoustic alarm signals.

According to the invention, the portable negative pressure wound therapy device further comprises an impact sensor 29, which is capable of communicating with electronic control unit 18. As explained above, the electronic control unit 18 comprises a microcontroller. The impact sensor 29 is one or more selected from the group of linear acceleration sensors, gyration sensors, humidity sensors, temperature sensors, magnetic field sensors and atmospheric air pressure sensor. Preferably, the impact sensor 29 is a linear acceleration sensor, optionally in combination with a gyration sensor and/or a magnetic field sensor.

It is possible, that the impact sensor 29 communicates directly, by means of an interface, with the electronic control unit 18. In this embodiment (herein called an "integrated impact sensor"), the impact sensor 29 is an integrated component of the control system 18 of the device. Also, the impact sensor data are processed by the electronic control unit 18 and the sensor raw data and/or the processed data storage is controlled by the electronic control unit 18. The integrated impact sensor 29 is usually (but not necessarily) provided with electric energy by the main power supply system of the device.

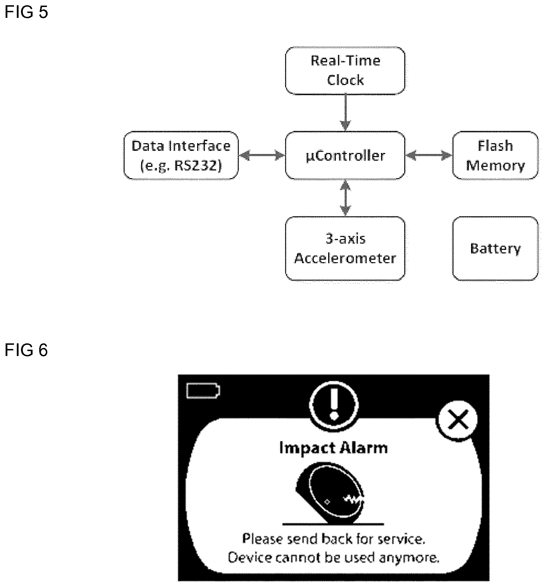

More preferably, the impact sensor 29 is a component of a stand-alone impact sensor module. A stand-alone impact sensor module is explained in more detail in FIG 5 below.

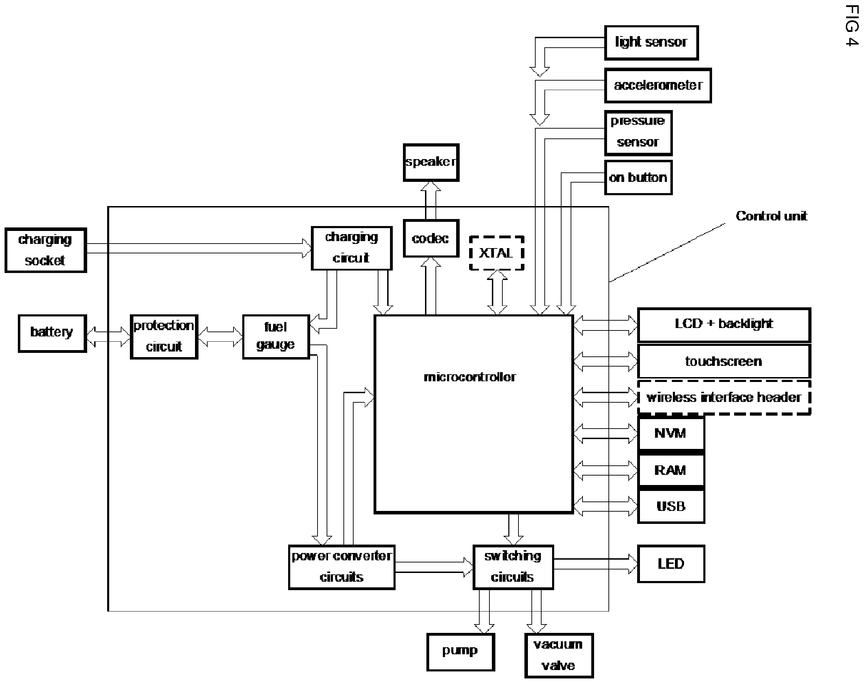

Interaction of the impact sensor 29 with the control unit 18 is also shown in FIG 4 below.

The impact sensor 29 can conveniently be mounted to the same main board, on which electronic control unit 18 is sitting.

Proceeding to FIG 4, communication of the control unit with peripheral components is shown schematically. One peripheral component communicating with the controller is the integrated impact sensor (termed "accelerometer" in FIG 4). Instead of an integrated impact sensor a stand-alone impact sensor module can be used likewise.

The control unit includes elements/functions such as the microelectronic controller ("microcontroller"), a protection circuit for the power supply, a fuel gauge for the power supply, a charging circuit for the power supply, power converter circuits, a speaker controller and switching circuits for controlling the suction pump and the electromagnetic valve.

The peripheral electric/electronic components include a source of electric energy, preferably a rechargeable battery, the suction pump, preferably a membrane pump and at least one electronic memory typically including both ROM and RAM chips. In up-to date devices, the peripheral electric/electronic components may additionally include, for example, a vacuum valve for a rinsing fluid pathway (as exemplified in FIG 3), interfaces such as a USB connector, electric connectors, such as a connector for the battery charger, a light sensor, a pressure sensor for sensing the pressure within the closed fluid-system of the device, a speaker for signalling warnings to the user, one or more LEDs for signalling warnings and/or status information to the user, a display, preferably a touchscreen display for interacting with the user, a backlight for the display, buttons, such as an on/off button. More advanced peripheral components such as a wireless interface for communication of the control unit with a remote computer may optionally be present.

The control unit is the central instance for controlling and monitoring function of the device.

According to the invention the peripheral components include an impact sensor, which may be an integrated impact sensor or preferably a stand-alone impact sensor module, each of which communicate with the microcontroller via an interface. The microcontroller receives impact sensor data. If an integrated impact sensor is used, the microcontroller receives the sensor raw data, for example acceleration data or temperature data. The raw data are processed as explained in detail above. If a stand-alone impact sensor module is used, the microcontroller receives the processed data and may, for example, display warnings to the user or lock the device.

The stand-alone impact sensor module shown in FIG 5 comprises at least one impact sensor and a further microelectronic controller (in the following called "impact sensor module controller"; in FIG 5 the impact sensor module controller is shortly termed "μController") cooperating with the impact sensor. The impact sensor module controller functions independent of the microelectronic controller of the control unit. The module further comprises a data interface, such as a RS232 interface, a memory, such as a flash memory and a real-time clock. The sensor, the interface, the memory and the real-time clock are in direct electronic communication with the impact sensor module controller. A separate battery or accumulator supplies electric energy to the components of the module thus making the module independent of the charging status of the main battery of the device. According to a particularly preferred embodiment of the invention, the module comprises at least one linear acceleration sensor, capable of detecting crash impacts. In practice, the sensor capable of detecting crashes should comprise at least an integrated linear acceleration sensor chip capable of detecting acceleration in the three axis of space ($a_x$; $a_y$; $a_z$). A particular advantage of using a stand-alone impact sensor module is, that the module can function independently of the working status of the device. The stand-alone impact sensor module can be active even if the device is switched off, as described in connection with the preferred embodiments herein. Thus, the stand-alone impact sensor module may perpetually report detrimental impacts that were acting on the device, for example also during storage of the device or during transport of the device.

FIG 6 shows an example of a warning message that is signalled to the user by means of a LCD-display. The alarm message informs the user that an impact was acting on the device and furthermore that the device is locked. Unlocking of the device can only be accomplished by a service technician authorised by the supplier/manufacturer. Before unlocking the device the service technician will perform a careful inspection of the device, optionally in combination with a prophylactically exchange of components, which are typically prone to deficiency after an impact of the type reported by the impact sensor. Hereby the service technician can also utilise information about the strength of the impact, which is stored in the memory of the device.

EXAMPLE 1

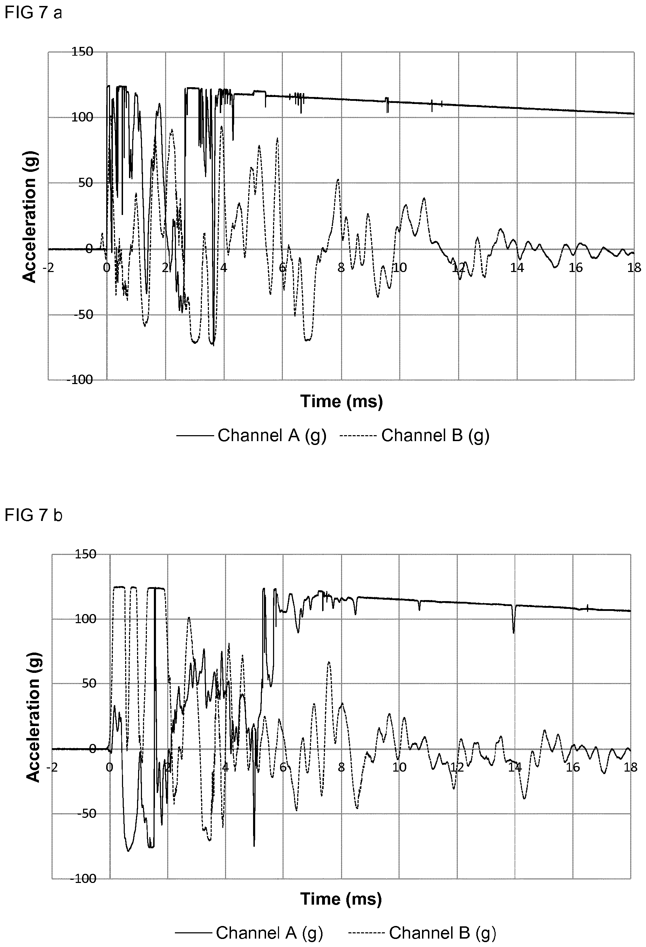

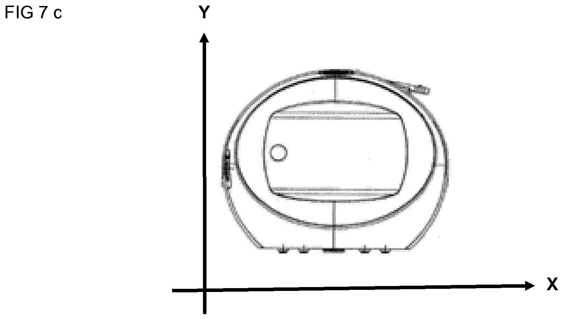

FIG 7 a and b show examples of unprocessed acceleration sensor data (g-forces) recorded from an linear acceleration sensor mounted to the main board of a portable negative pressure wound therapy device (VivanoTec® negative pressure unit from Paul Hartmann AG, Germany). The data corresponding to FIG 7 a were received while the device was subjected to a drop test. During the test, the device was dropped to a hard surface (metal plate) from a height of 0,4 m above ground. In this experiment, as detected by a high speed camera running at 2000 fps, the device crashed to the bottom front face.

FIG 7b shows another experiment, where the device crashed to the side face. The acceleration values corresponding to the g-forces along the two axes x and y (see FIG 7 c) are displayed in the diagram. Each of the x- and y-axis sensors detected maximum acceleration values of more than 120 g in both tests. The g-force peak occurs in less than 1 ms. The resolution of the sensor was set to 1000 Hz. Therefore, the actual acceleration peak values could have been even higher. Peaks may escape detection, if occurring in a time interval much smaller than the timely resolution of the sensor. Drops of the type described in these examples (according to FIG 7 a and b) do not leave any visible damages to the device in most cases. However, the acceleration occurring during the drop can potentially impose stress to internal mechanical or electronic components, which may lead to a malfunction during future therapy cycles. It is a significant advantage of the invention that the impact sensor is capable of warning a user of the device of any "sleeping" malfunction that may wake up during later usage of the device. Therefore, a portable negative pressure wound therapy device according to the invention is more reliable and safer.

EXAMPLE 2

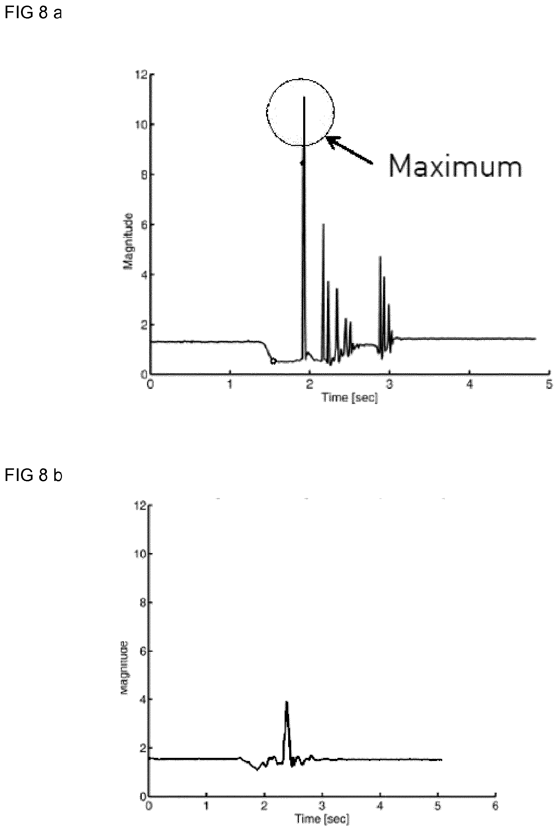

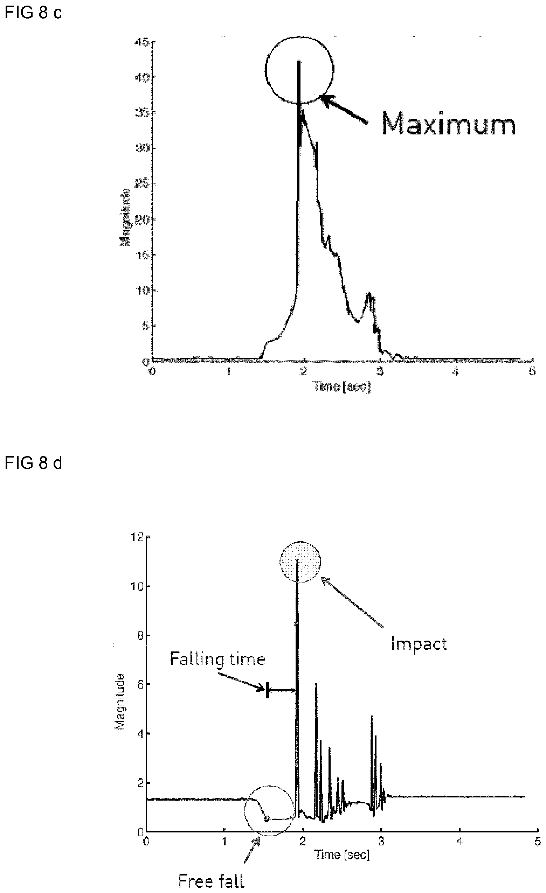

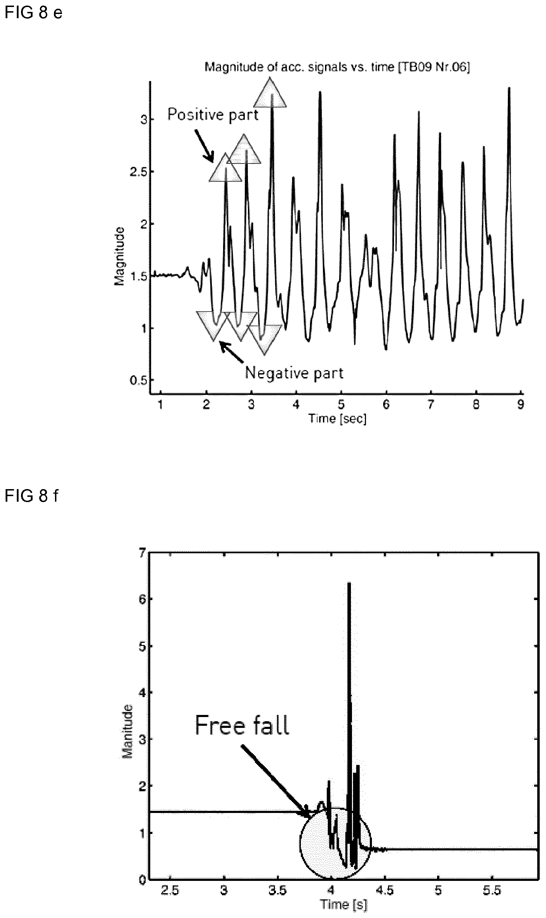

FIG 8 a shows examples of processed impact sensor data recorded by means of an inertial sensor module mounted to the main board of a portable negative pressure wound therapy device (VivanoTec® negative pressure unit from Paul Hartmann AG, Germany). For the experiments the IMU motion sensor LSM9DS0 ("iNEMO inertial module") from ST Microelectronics, United Kingdom was used. This inertial module device has three acceleration channels, three angular rate channels and three magnetic field channels. Data from the magnetic field channels were not recorded.

The data corresponding to FIG 8 a were received while the device was subjected to a drop test. During the test the device was dropped to a soft ground (rubber plate) from a height of 1,0 m above ground. A maximum magnitude $M_\alpha$ value > 11 (relative units) was observed. A drop of this kind usually does not leave any visible damages to the device. However, the acceleration occurring during the drop can potentially impose stress to internal mechanical or electronic components, such as electric contacts or tube connections, which may lead to a malfunction during future therapy cycles.

The data corresponding to FIG 8b were received while the same VivanoTec® device was carried on the body of a moving test user. During the recording the user was changing his position from an upright (standing) position to sitting down to a chair. The movement of the test user generated acceleration signals detected by the impact sensor. A maximum magnitude $M_\alpha$ value of about 4 (relative units) was observed. However, such comparatively low acceleration forces will not cause any detrimental stress to the device. An impact of such kind must not cause any warning signals to the user.

The diagrams show the magnitude $M_\alpha$ calculated from the recorded outputs of the three linear acceleration sensors included in a MEMS motion sensor (H3LIS331 DL from ST Sensor Technology, United Kingdom). $M_\alpha$ was calculated as a relative value using the formula

$$M_a = \sqrt{(a_x^2 + a_y^2 + a_z^2)}$$

wherein the values ($a_x$; $a_y$; $a_z$) correspond to the output of the sensor channels measuring linear acceleration along the three space axis (x; y; z).

Suitable predetermined impact thresholds, for example impact thresholds for acceleration, can be set by repeatedly performing experiments of the type described in this example. The impact thresholds for acceleration will have to be determined for each type of device, because they depend on device specific parameters like stability, weight, material and shape of the device. The data displayed in this example indicate, that an adequate threshold will have to be above the non-detrimental impact (device carried by user while changing his position from standing to sitting, whereby a maximum magnitude $M_\alpha$ value of 4 relative units was observed) and below the potentially detrimental impact (dropping the device from a height of 1 m to ground, whereby a maximum magnitude $M_\alpha$ value of about 11 relative units was observed). In order to set adequate thresholds, a series of experiments of the type described in this example will have to be carried out.

Fig 8c shows the magnitude $M_\omega$ reflecting the overall gyration forces that the device was exposed to during the same experiment (drop to a soft ground) already described under FIG 8 a. A maximum magnitude $M_\omega$ value of > 42 (relative units) was observed. A comparison of FIG 8 a with FIG 8 c shows that the peak of acceleration magnitude $M_\alpha$ coincides with the peak of gyration magnitude $M_\omega$. This indicates that the crash entails both strong linear acceleration and strong rotation forces, which occur simultaneously.

FIG 8d again shows the graph of FIG 8 a providing additional information about the incidents occurring during curve progression. The free fall phase of the experiment, during which gravity approaches towards zero (therefore minimising magnitude $M_\alpha$, if the device is dropped vertically) is indicated by a first circle. The free fall ends with the impact to the ground leading to the acceleration peak. After the drop, the height of the fall can be estimated, using the recorded acceleration values and a standard function analysis algorithm. The algorithm is adapted to detect the begin of the free fall ($t_{ff}$; first downward turning point of the acceleration function) and the begin of the impact ($t_{im}$, first upward turning point of the acceleration function). The height of the fall can then be calculated using the formula

$$s \text{ (height)} = g \text{ (gravity)} \times (t_{im} - t_{ff})^2$$

For the estimation of the height of the fall the air resistance is disregarded. In the case shown in FIGs 8 a/d the time span of the free fall (taken from the diagram) is about 0,33 s leading to an estimated drop height of 1,07 m [9.81 m/s$^2$ x (0,33 s)$^2$= 1,07 m], which is a good approximation to the actual value of 1.00 m.

FIG 8e shows the magnitude $M_\omega$, which was recorded while the device was carried on the body of a moving test user, who went down a stair. All observed peaks of magnitude $M_\omega$ are below 3,5 (relative units) und thus remain in a similar range as the peaks observed in the experiment shown in FIG 8 b. Comparatively higher values (as compared to the "walking down the stairs") of magnitude $M_\omega$ (more than 6) were detected, when the device was flipped over to the face side (FIG 8 f). However, the relative magnitude $M_\omega$ values related to the flip are still far below the relative magnitude $M_\omega$ values caused by a drop as described in accordance with FIG 8 a (more than 11). The VivanoTec® device used for the experiments described in this example 2 would not suffer any detrimental effects after a flip.

The experiments shown in connection with this example 2 demonstrate, that an acceleration threshold, for example a threshold of 8 relative units for the exemplified case, can conveniently be determined by subjecting the device to a series of movement and crash conditions, which are non-detrimental or detrimental for the device, respectively. Exceeding of the threshold can serve as trigger, for example, to record impact sensor data on a memory, to provoke a warning message, to lock the device, to send a message to a remote server (if a remote interface is present) or to increase sampling rate of the sensor. It is also possible to use several (for example two or three) different thresholds resulting in different activities, if the threshold is exceeded. On preferred example is to use a lower threshold, for example 2 relative units to activate the sleep-to-wakeup function and to use a comparatively higher threshold, for example 8 relative units to display a warning message and/or lock the device. Examples for threshold dependent activities are described more in detail in the description of specific preferred embodiments above.

## Claims

1. Method of recording an impact condition, to which a negative pressure therapy device (1) was exposed, comprising the steps

   - providing a portable negative pressure wound therapy device (1) comprising

      - an electrically actuated suction pump (5) for draining wound fluids from a patient,
      - at least one microelectronic controller,
      - at least one electronic memory,
      - a housing (4) for containing the electrical and/or electronic components,
      - at least one microelectronic impact sensor (29), wherein the impact sensor (29) is adapted to detect an impact acting on the device (1),

   - setting at least a first predetermined impact threshold,
   - setting a second predetermined impact threshold,
   - detecting an impact by means of the impact sensor (29),
   - the microelectronic controller recording the impact, if the impact detected by the impact sensor (29) is above the first predetermined threshold,
   - further comprising the steps of the microelectronic controller alarming an user and/or locking the negative pressure wound therapy device (1), if the impact detected by the impact sensor (29) is above the second predetermined threshold, the second predetermined threshold being higher than the first predetermined threshold.

2. Method according to claim 1, wherein the impact is linear acceleration and/or rotation.

## Patentansprüche

1. Verfahren zum Aufzeichnen eines Einwirkungszustands, dem eine Unterdrucktherapievorrichtung (1) ausgesetzt war, umfassend die folgenden Schritte:

   Bereitstellen einer tragbaren Unterdruck-Wundtherapievorrichtung (1), die Folgendes umfasst:

      eine elektrisch ausgelöste Saugpumpe (5) zum Ablassen von Wundflüssigkeiten von einem Patienten,
      mindestens eine mikroelektronische Steuerung,
      mindestens einen elektronischen Speicher,
      ein Gehäuse (4) zum Aufnehmen der elektrischen und/oder elektronischen Komponenten,
      mindestens einen mikroelektronischen Einwirkungssensor (29), wobei der Einwirkungssensor (29) dazu ausgebildet ist, eine auf die Vorrichtung (1) agierende Einwirkung zu detektieren, Einstellen zumindest einer ersten vorbestimmten Einwirkungsschwelle,

   Einstellen einer zweiten vorbestimmten Einwirkungsschwelle,
   Detektieren einer Einwirkung mittels des Einwirkungssensors (29),
   wobei die mikroelektronische Steuerung die Einwirkung aufzeichnet, falls die durch den Einwirkungssensor (29) detektierte Einwirkung über der ersten vorbestimmten Schwelle liegt, ferner umfassend die Schritte, dass die mikroelektronische Steuerung einen Benutzer alarmiert und/oder die Unterdruck-Wundtherapievorrichtung (1) sperrt, falls die durch den Einwirkungssensor (29) detektierte Einwirkung über der zweiten vorbestimmten Schwelle liegt, wobei die zweite vorbestimmte Schwelle höher ist als die erste vorbestimmte Schwelle.

2. Verfahren nach Anspruch 1, wobei die Einwirkung eine Linearbeschleunigung und/oder Rotation ist.

## Revendications

1. Procédé d'enregistrement d'un état d'impact auquel a été exposé un dispositif de thérapie par pression négative (1), comprenant les étapes suivantes :

   - fourniture d'un dispositif portatif de thérapie de plaies par pression négative (1), comprenant :

      - une pompe d'aspiration à commande électrique (5) pour drainer des fluides d'une plaie d'un patient,
      - au moins un dispositif de commande microélectronique,
      - au moins une mémoire électronique,
      - un boîtier (4) pour contenir les composants électriques et/ou électroniques,
      - au moins un capteur d'impact microélectronique (29), le capteur d'impact (29) étant prévu pour détecter un impact agissant sur le dispositif (1),

   - régler au moins un premier seuil d'impact prédéterminé,
   - régler un deuxième seuil d'impact prédéterminé,
   - détecter un impact au moyen du capteur d'impact (29),

- le dispositif de commande microélectronique enregistrant l'impact, si l'impact détecté par le capteur d'impact (29) est au-dessus du premier seuil prédéterminé,

- comprenant en outre les étapes de déclenchement d'une alerte, par le dispositif de commande microélectronique, pour un utilisateur, et/ou de verrouillage du dispositif de thérapie de plaies par pression négative (1), si l'impact détecté par le capteur d'impact (29) est au-dessus du deuxième seuil prédéterminé, le deuxième seuil prédéterminé étant plus élevé que le premier seuil prédéterminé.

2. Procédé selon la revendication 1, dans lequel l'impact est une accélération linéaire et/ou une rotation.

FIG 1

FIG 2

Fig. 2a

Fig. 2b

Fig. 2c

Fig. 2d

Fig. 2e

FIG 3

FIG 4

FIG 5

FIG 6

FIG 7 a

Channel A (g)   Channel B (g)

FIG 7 b

Channel A (g)   Channel B (g)

FIG 7 c

FIG 8 a

FIG 8 b

FIG 8 c

FIG 8 d

FIG 8 e

Magnitude of acc. signals vs. time [TB09 Nr.06]

FIG 8 f

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040073151 A1
- WO 2009047524 A2
- EP 1905465 A1
- WO 2008039314 A2
- EP 777504 B1
- EP 1863549 B1
- EP 2464394 A1
- WO 2012156174 A1
- EP 2464393 A1
- EP 0777504 B1
- US 20150025485 A1
- US 20100305523 A1
- WO 2002093272 A1
- US 2015073338 A1